# EUROPÄISCHE PATENTSCHRIFT

(11) **EP 1 157 272 B1**
(45) Veröffentlichungstag und Bekanntmachung des Hinweises auf die Patenterteilung: **20.08.2003**
(21) Anmeldenummer: 00914076.5
(22) Anmeldetag: 09.02.2000
(51) Int. Cl.: G01N 33/28, G01N 27/12, G01N 27/22

(54) **ÖLQUALITÄTSSENSOR SOWIE VERFAHREN ZU DESSEN HERSTELLUNG**
OIL QUALITY SENSOR AND METHOD FOR PRODUCING THE SAME
CAPTEUR DE QUALITE D'HUILE ET SON PROCEDE DE FABRICATION

(30) Priorität: 23.02.1999 DE 19907743
(43) Veröffentlichungstag der Anmeldung: 28.11.2001
(73) Patentinhaber: Volkswagen Aktiengesellschaft, 38436 Wolfsburg (DE)
(72) Erfinder: DICKERT, Franz, Ludwig, A-3400 Klosterneuburg (AT)
(86) Internationale Anmeldenummer: EP0001021
(87) Internationale Veröffentlichungsnummer: WO00050894

(56) Entgegenhaltungen:
- WO-A-98/19156
- US-A- 4 731 421
- US-A- 5 050 434
- US-A- 5 136 274
- US-A- 5 161 085
- US-A- 5 177 662
- US-A- 5 332 961
- US-A- 5 408 381

## Beschreibung

Die Erfindung betrifft einen Ölqualitätssensor gemäß dem Oberbegriff des Anspruchs 1 sowie ein Verfahren zur Herstellung der sensitiven Schicht eines solchen Ölqualitätssensors.

Motorenöle haben sich im Laufe der Zeit zu High-Tech-Produkten entwickelt, die mit Vorraussetzung sind, hohe Motorleistungen zur ermöglichen. "Motorenöle" stellen dabei eine Sammelbezeichnung für Grundölkomponenten aus Mineralöl, Hydrocrackanten und synthetischen Komponenten dar. Motorenöle enthalten noch Additive, die als Fertigmischung ("Paket") zugegeben werden, sowie Viskositätsindex-Verbesserer (VI). Die Motorenöle dienen dabei als Schmierstoffe für die Motoren sowie als Kühl- und Abdichtmedium. Weiterhin sollen sie sämtliche Motorenteile reinigen und reinhalten. Die VI-Verbesserer bewirken ein günstigeres Viskositäts-Termperaturverhalten, als es die reinen Grundöle aufweisen. Der Anteil an Additiven und VI-Verbesserer liegt je nach Anforderungen, die an das Öl gestellt werden, üblicherweise zwischen 5 und 25 %.

Weitere Aufgaben der Additive in den Motorenölen sind die Verbesserung der Korrosionsschutzeigenschaften der Öle sowie die Verhinderung von Schlammablagerungen und Öleindickungen sowie der Verschleißschutz an den Reibpartnern unter allen auftretenden Belastungen. Die thermischen Beanspruchungen der Motorenöle sind hoch und liegen dabei im Ölsumpf durchschnittlich bei ca. 100 bis 150 °C. Im Bereich der oberen Kolbenringzone können Temperaturspitzen zwischen 200 und 350 °C auftreten.

Im Laufe ihres Gebrauchs altern die Öle, wobei in erster Linie die Additivkomponenten und die VI-Verbesserer abgebaut (verbraucht) werden. Einen erheblichen Anteil an der Öltalterung haben unverbrauchte, teilweise oxidierte und polymerisierte Ölkomponenten. Somit wird die Ölalterung durch Temperatureinwirkung und reaktionsfähige Verbrennungsprodukte (Radikale) sowie über das Überschreiten der Dispergierfähigkeit der Öle für Feststoffe und Alterungsprodukte hervorgerufen. Hierdurch werden dann die notwendigen Eigenschaften der Öle zum störungsfreien Betrieb der Motoren mitunter drastisch verschlechtert. Eine erhöhte Viskosität hat z.B. beim Startvorgang einen verlängerten Transport des Öles zu den Schmierstellen zur Folge, wodurch ein Anstieg des Verschleißes eintritt.

Der Verbrauch der Dispergieradditive hat eine Verschlechterung der Fähigkeit der Öle zur Reinhaltung der Motoren, insbesondere an kritischen Schmierstellen, wie im Bereich der Kolbenringe/Nuten und Feuerstege, zur Folge, sowie eine Verschlechterung bei der Verhinderung der Ablagerungsbildung an Ventilen und im Ventiltrieb.

Wünschenswert ist es daher, die während des motorischen Betriebes zwangsläufig auftretende Verschlechterung der Eigenschaften der Motorenöle kontinuierlich oder in kurzen Zeitabschnitten, d.h. beispielsweise ein oder mehrmals während eines Betriebes einer Brennkraftmaschine, zu erfassen.

Bis heute ist es jedoch noch nicht gelungen, zuverlässige Meßfühler für eine Ölzustandsanalyse zu entwickeln, wobei für einen längeren Betrieb des Motorenöls im Motor, insbesondere bei instationären Motoren, eine on-board-Analyse, d.h. eine Analyse direkt am Motor, erforderlich ist.

Bisher wurden die unterschiedlichsten Ölsensoren entwickelt, die insbesondere die Viskosität, TAN (Total Acid Number) oder den Füllstand sensieren. Eine besondere Schwierigkeit ist hierbei die Verwendung unterschiedlicher Öle an ein und derselben Brennkraftmaschine sowie das Kompensieren unterschiedlicher Alterungseinflüsse auf die sensierte Eigenschaft. So ist es beispielsweise aus US-A 4,675,662 und 4,721,874, EP 527 176 B und JPN. Appl. Phys., 1993, Acoustic Plate Viskosity Sensor, bekannt, die mit der Alterung des Öls geänderte Viskosität als Meßgröße für den Ölzustand heranzuziehen. Dies geschieht über akustische Laufzeitveränderungen, die Phasenverschiebung oder über Eigenfrequenzänderungen eines Schwingquarzes. Problematisch sind hierbei zum einen die häufig fehlende Möglichkeit, die Messung on-board vorzunehmen, und zum anderen die möglichen gegenläufigen Effekte "Abbau des Motorenöls und Verdünnung durch Kraftstoff", die die Viskosität erniedrigen, gegenüber der "Verknüpfung der Abbauprodukte", die die Viskosität erhöhen, solange sie nicht als Schlamm ausfallen.

Die TAN oder auch TBN (Total Basic Number) ist vom Grundprinzip für eine on-board-Messung nicht geeignet, da hierbei das Altöl mit KOH titriert wird. Neuere Ansätze, wie beispielsweise aus SAE 910497, SAE 962112, US-A 4,675,662, 4,792,791 und 5,200,027 bekannt, zeigen hier interessante Lösungen, die beispielsweise mit Kapazitätssensoren, Messung der lonenwanderung oder einer Potentialdifferenz, mit elektrochemischen Festkörperzellen und mit Korrosionssensoren arbeiten. Diese Lösungsansätze sind teils ungenau, noch zu groß und zu schwer oder benötigen ein Opferbauteil, das grundsätzlich unerwünscht ist. Ferner sind noch mathematische Modelle (SAE 870403) und HC-Abgassensoren (DE 42 35 225) bekannt, die bislang auch noch nicht zu einem Durchbruch geführt haben. Auch Füllstandssensoren sind wenig geeignet, da diese beispielsweise bei einer starken Verdünnung des Motorenöls durch Kraftstoffe versagen.

In "Molekular Imprinting of Chemically Sensitive Coatings - New Strategies for Sensor Design and Fabrication -l" von F. L. Dickert, P. Forth, P. Lieberzeit, M. Tortschanoff, W.-E. Bulst, U. Knauer und G. Fischerauer in "Sensor 97" 8. internationale Messe, Nürnberg, 1997, wird ein molekulares Prägen für massensensitive Sensorik in Gasen wie in Flüssigkeiten näher beschrieben.

Ein Ölqualitätssensor zur Bestimmung der Ölqualität von z.B. Motorenölen wird in der DE 197 31 621.2 A1 beschrieben. Dort wird unter anderem ausgeführt, daß die Alterung eines Öls unter den Bedingungen einer Brennkraftmaschine mit einer steigenden Acidität einhergeht, d.h., daß eine Zunahme acider Verbindungen festgestellt werden kann. Deshalb wird in dieser Schrift vorgeschlagen, die sensitive Schicht so auszubilden, daß sie sich für einen Nachweis der als Abbauprodukte des Öls entstehenden aciden Verbindungen eignet. Dazu werden für den Ölqualitätssensor Polyurethane eingesetzt, die durch den Einbau basischer Komponenten in die Polymerstruktur modifiziert werden.

Aufgabe der vorliegenden Erfindung ist es, einen Ölqualitätssensor zur Verfügung zu stellen, dessen sensitive Schicht aufgrund ihrer chemischen Zusammensetzung für die Bedingungen, bei denen der Ölqualitätssensor eingesetzt wird, insbesondere an einer Brennkraftmaschine, eine besondere Eignung aufweist.

Die Lösung dieser Aufgabe liefert ein erfindungsgemäßer Ölqualitätssensor mit den kennzeichnenden Merkmalen des Anspruchs 1.

Umfangreiche Untersuchungen im Rahmen der vorliegenden Erfindung haben gezeigt, daß sich besonders geeignete resistente Ölqualitätssensoren herstellen lassen, wenn die sensitive Schicht wenigstens ein Polyimid oder Polyamid umfaßt. Vorzugsweise ist das erfindungsgemäße Polyamid bzw. Polyimid der sensitiven Schicht durch Polykondensationsreaktionen erhältlich ausgehend von wenigstens einem Carbonsäureanhydrid und wenigstens einem zwei- oder mehrfunktionellen Amin. Weiter vorzugsweise geht man bei der Polykondensationsreaktion aus von einem mehrfunktionellen Carbonsäureanhydrid und wenigstens einem zwei- oder mehrfunktionellen Amin.

Besonders geeignete Polyamide oder Polyimide für die sensitive Schicht eines Ölqualitätssensors gemäß der Erfindung sind erhältlich in einer wenigstens zweistufigen Reaktion, wobei in der ersten Reaktionsstufe auf vorzugsweise zwischen 60° und 90° C erwärmt wird zum Starten der Polyamidbildung und weiters dann auf vorzugsweise zwischen 130° und 170° erhitzt wird zur Entfernung des Lösungsmittels und Vervollständigung der Polyamidbildung. In der zweiten Reaktionsstufe wird auf Temperaturen über 200° erhitzt um die Polyimide zu bilden.

Weiterhin setzt man vorzugsweise für die Herstellung der sensitiven Schicht des Ölqualitätssensors wenigstens drei Monomerkomponenten ein, nämlich vorzugsweise wenigstens ein mehrfunktionelles Carbonsäureanhydrid, wenigstens ein difunktionelles Amin und wenigstens ein trifunktionelles Amin. Als mehrfunktionelles Carbonsäureanhydrid kommt beispielsweise Pyromellitsäuredianhydrid in Betracht. Für die difunktionelle Aminkomponente ist beispielsweise Phenylendiamin besonders geeignet. Vorzugsweise ist die sensitive Schicht des Ölqualitätssensors erhältlich ausgehend von wenigstens einem aliphatischen Amin als Aminkomponente. Besonders bevorzugt ist es, daß man zur Herstellung der sensitiven Schicht des Ölqualitätssensors von wenigstens einem trifunktionellen aliphatischen Amin als Aminkomponente ausgeht. Der Anteil der Triaminkomponente liegt vorzugsweise zwischen etwa 40 % und etwa 80 %.

Als vorteilhaft hat es sich erwiesen, wenn bei der Herstellung des Polyamids oder Polyimids für die sensitive Schicht ein Überschuß wenigstens einer der Aminkomponenten in der Monomermischung eingesetzt wird.

Besonders geeignete Verbindungen für die trifunktionelle Aminkomponente, die bei der Polymerisation als Vernetzer wirken kann, sind z.B. Triaminopyrimidin und/oder 4-Aminomethyl-1,8-diaminooktan.

Die Erfindung betrifft weiterhin ein Verfahren zur Herstellung der sensitiven Schicht eines Ölqualitätssensors der vorgenannten Art. Vorzugsweise geht man zur Herstellung eines erfindungsgemäßen Ölqualitätssensors so vor, daß man die Monomerkomponenten mischt, dann das interessierende Öl zugibt um die sensitive Schicht zu prägen und dann diesen Ansatz mindestens so hoch erhitzt, daß die Reaktion startet. Vorzugsweise erhitzt man die nach einer ersten Reaktionszeit entstehende homogene Lösung, die z.B. auf einen für eine FT-IR-Messung geeigneten Preßling aufgebracht oder auf einen Schwingquarz aufgebracht wurde danach eine Zeit lang bei einer höheren Reaktionstemperatur in einer ersten Reaktionsstufe. Weiter beträgt vorzugsweise in einer ersten Reaktionsstufe die Temperatur, auf die man erhitzt, um die Amidbildung zu starten, etwa 60° bis 90° C und danach zur Entfernung des Lösungsmittels und Vervollständigung der Reaktion etwa 130° bis 170°. Besonders bevorzugt ist es, daß danach in einem weiteren Schritt das Polyamid der sensitiven Schicht auf dem Träger, z.B. dem Preßling oder dem Schwingquarz zur Imidbildung auf eine noch höhere Temperatur erhitzt wird, beispielsweise auf eine Temperatur zwischen 230° und 270° C, vorzugsweise auf eine Temperatur von etwa 250°.

Bei dem erfindungsgemäßen Verfahren zur Herstellung der sensitiven Schicht eines Ölqualitätssensors stellt man vorzugsweise zu Beginn eine Lösung der Monomeren in N-Methyl-pyrrolidon her, beispielsweise in einer bevorzugten Konzentration von zwischen etwa 0,05 mol/l und 0,2 mol/l. Das interessierende Öl gibt man vorzugsweise zu der fertigen Mischung der Monomeren in einer bezogen auf die Monomerkomponenten etwa 1,3 bis 1,7 fachen Gewichtsmenge, vorzugsweise in einer etwa 1,5 fachen Gewichtsmenge.

Die in den Ansprüchen 2 bis 12 angegebenen Merkmale betreffen bevorzugte Ausführungsformen des erfindungsgemäßen Ölqualitätssensors. Die Ansprüche 14 bis 18 betreffen bevorzugte Varianten des Verfahrens zur Herstellung der sensitiven Schicht des erfindungsgemäßen Ölqualitätssensors.

Laut Literatur, siehe u. a. H.-G. Elias "An Introduction to Polymer Science", 1. Aufl., VCH Weinheim, 1997, 133 f, können Polyimide durch zweistufige Polykondensationsreaktionen von mehrfunktionellen Carbonsäureanhydriden (zum Beispiel Pyromellitsäuredianhydrid) und zwei- bzw. mehrfunktionellen Aminen hergestellt werden. Die Polymere werden am Ende eines zweistufigen Reaktionsverlaufs bei 250°C ausgehärtet. Dabei verdampft das Solvens und es bildet sich unter Wasserabspaltung das Polyimid, das hoch temperaturstabil ist (teilweise bis 500°C beständig).

Für die Verwendung als Ölsensor müssen in dem Material adaptierte Hohlräume generiert werden, die fähig sind, Motorenöle abhängig von ihrem Verbrauchszustand selektiv einzulagern um damit ein meßbares Signal für die Ölalterung zu liefern. Der große Vorteil der Polyimide gegenüber anderen Polymeren liegt in erster Linie in deren hoher thermischer Beständigkeit, aber auch in dem Vorhandensein polarer Gruppen, die eine selektivere Anpassung an die zunehmende Polarität des Altöls ermöglichen. Zum Prägen des Polyimids wird das Öl in der Regel direkt mit den Monomeren und dem notwendigen Lösungsmittel gemischt. Im Zuge der fortschreitenden Polyamidbildung bildet sich eine homogene Lösung.

Die Ein- und Auslagerung der Motorenöle wird im Rahmen der Erfindung vorzugsweise mit Hilfe der FT-IR-Spektroskopie verfolgt. Als Maß für den Ölgehalt kann z.B. die Absorbanz der aliphatischen Methylenstreckschwingungen bei 2856 cm⁻¹ bzw. 2921 cm⁻¹ dienen.

Aufgrund Ihrer Stabilität sind Polyamide bzw. Polyimide der erfindungsgemäßen Art für Anwendungen in Motoren und den dabei auftretenden thermischen und chemischen Belastungen sehr gut für sensorische Anwendungen in der Ölanalytik geeignet.

Nachfolgend wird die vorliegende Erfindung anhand von Beispielen unter Bezugnahme auf die beiliegenden Figuren näher beschrieben. Dabei zeigen
Fig. 1 ein Diagramm in dem die Abhängigkeit der Wiedereinlagerung von Neuöl in Polymere mit variablem Vernetzeranteil dargestellt ist;
Fig. 2 ein Diagramm das die Abhängigkeit der Selektivität einer Neuöl-geprägten Polyimidschicht in Abhängigkeit vom Vernetzeranteil zeigt;
Fig. 3 ein Diagramm das die Abhängigkeit der Selektivität eines Neuölimprints in Abhängigkeit vom Überschuß an Aminokomponente bezogen auf das Polyimid zeigt;
Fig. 4 ein Diagramm das die Abhängigkeit der Selektivität eines Polyamids (80 % Triamin) von der Vorpolymerisationsdauer zeigt.

### Beispiele

### Arbeitsvorschrift

Als Monomerkomponenten dienen Pyromellitsäuredianhydrid, 4-Aminomethyl-1,8-diaminooktan, Phenylendiamin sowie Triaminopyrimidin. Von allen trifunktionellen Monomeren werden Lösungen der Konzentration 0,1 mol/l in N-Methyl-pyrrolidon hergestellt, bei den difunktionellen beträgt die Konzentration 0,15 mol/l. Das entspricht für die einzelnen Komponenten folgenden Einwaagen:

| | | |
|---|---|---|
| Pyromellitsäure-dianhydrid | difunktionell | 32,7 mg/ml |
| Phenylendiamin | difunktionell | 10,8 mg/ml |
| Triaminopyrimidin | trifunktionell | 12,5 mg/ml |
| 4-Aminomethyl-1,8-diaminooktan | trifunktionell | 18,7 mg/ml |

Je nach gewünschtem Anteil an trifunktionellem Amin (das als Vernetzer wirkt) ergeben sich folgende Mischungsverhältnisse der einzelnen Komponenten:

| Vernetzeranteil | Säureanhydridlösung [µl] | Diaminlösung [µl] | Triaminlösung [µl] |
|---|---|---|---|
| 0 % | 133.3 | 200 | 0 |
| 10 % | 140,0 | 180 | 20 |
| 20 % | 146,7 | 160 | 40 |
| 30 % | 153,3 | 140 | 60 |
| 40 % | 160,0 | 120 | 80 |
| 50 % | 166,7 | 100 | 100 |
| 60 % | 173,3 | 80 | 120 |
| 70 % | 180,0 | 60 | 140 |
| 80 % | 186,7 | 40 | 160 |
| 90 % | 193,3 | 20 | 180 |
| 100 % | 200,0 | 0 | 200 |

Zu den fertigen Mischungen wird das interessierende Öl zugegeben, wobei die zugesetzte Masse in etwa 1,5-mal höher ist als jene der Monomerkomponenten. Die so hergestellten Ansätze werden ca. 15 Minuten auf 75°C erhitzt um die Reaktion zu starten und zu beschleunigen. Die dabei entstehende homogene Lösung wird danach auf einen KBr-Preßling (für die FT-IR-Messungen) oder einen Schwingquarz aufgebracht und weitere 10 Minuten auf 150°C erhitzt. Diese Lösungen eignen sich auch ausgezeichnet für spin-coating-Verfahren. Schlußendlich bildet man das Polyimid durch einstündiges Erhitzen der Träger auf 250°C.

Obige Arbeitsvorschrift muß bei der Verwendung von 4-Aminomethyl-1,8-diaminooktan wegen dessen hoher Reaktivität etwas variiert werden. In diesem Fall wird mit allen übrigen Komponenten bis zur Auftragung der Reaktionsmischung auf den Träger gleich verfahren. Nach dem Verdunsten des Lösungsmittels bei 150°C bringt man die Aminlösung auf die entstehende teilweise polymere Schicht auf und läßt 10 Minuten bei 150°C aushärten. Die weitere Prozedur ist dann die selbe wie oben.

### Messungen und Diskussion

Die fertigen, beschichteten KBr-Preßlinge werden im Bereich zwischen 3500 cm⁻¹ und 700 cm⁻¹ untersucht. Als Maß für die Ein- und Auslagerung des Öls dient die IR-Absorption der aliphatischen CH₂-Banden bei 2856 cm⁻¹ bzw. 2921 cm⁻¹. Zudem kann bei Verwendung der Preßlinge der Verlauf der Polymerisationsreaktion verfolgt werden (der interessierende Bandenbereich liegt bei 1800-1500 cm⁻¹).

Bei der Überprüfung der untersuchten Vernetzer zeigt sich, daß vorzugsweise in die mit dem aliphatischen Amin hergestellten Schichten Prägeöl wieder eingelagert werden kann. Dies ist darauf zurückzuführen, daß es sich bei allen anderen Monomerkomponenten um Aromaten und damit um planare Moleküle handelt, deren Ringe sich möglichst parallel zueinander in geordneten Strukturen ausrichten. Die aliphatische Komponente ermöglicht eine größere sterische Flexibilität während der Polymerisation, ohne dabei allerdings die Struktur so weit aufzuweichen, daß sie nach der Entfernung des Öls kollabiert.

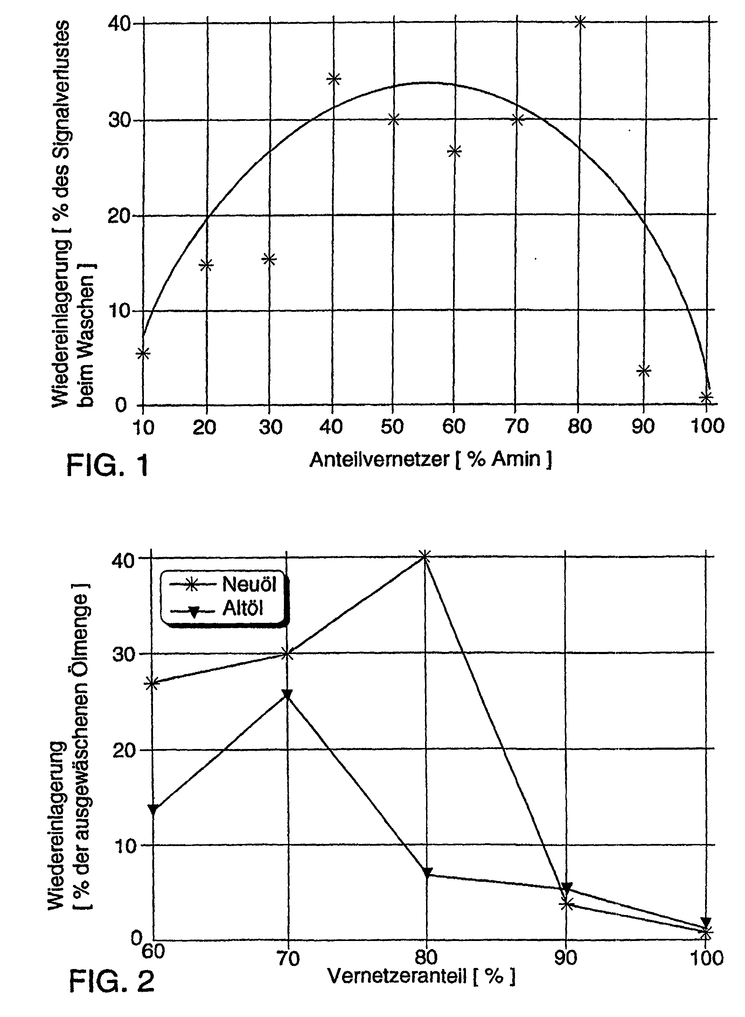

Fig. 1 zeigt für einen Imprint mit frischem Öl die Abhängigkeit der Wiedereinlagerung vom Vernetzeranteil. Man sieht deutlich, daß die Verwendung zu hoher und zu geringer Mengen an Triamin den Einschluß des Analyten unmöglich macht beziehungsweise stark erschwert. Ideal geeignet ist somit der Bereich zwischen 40% und 80% Triamin. Betrachtet man im Bereich höherer Vernetzeranteile die Selektivität der Schichten also den Unterschied in der Einlagerung von neuem und gebrauchtem Schmiermittel, so ergeben sich die in Fig. 2 gezeigten Zusammenhänge. Der größte Unterschied in der Einlagerung der beiden Öle wird demnach bei einem Vemetzeranteil von 80% erreicht.

Eine weitere Variationsmöglichkeit der Schichtchemie ergibt sich durch die Verwendung eines Überschusses an Aminokomponente in der Monomermischung, was je nach dessen Größe von einer Verminderung der Ringschlüsse zum Imid bis zu einem hoch vernetzten Polyamid ohne Imidbindungen reichen kann.

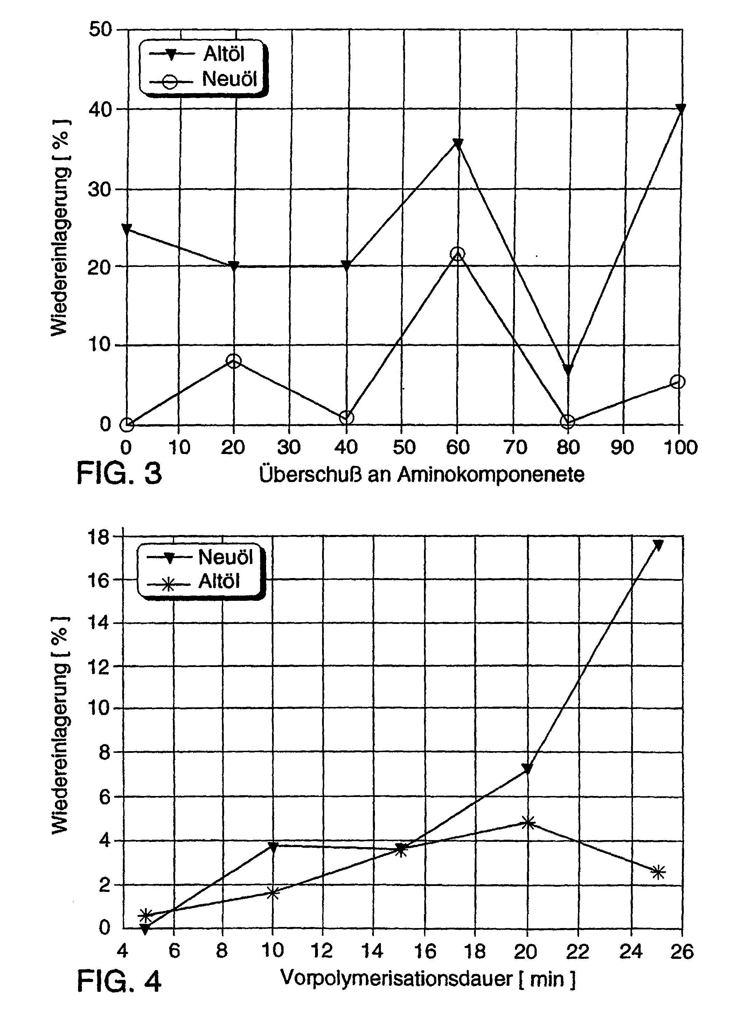

Der Vorteil der Synthese eines Polyamids liegt in der geringeren Starrheit der gebildeten Materialien im Vergleich zu den gebildeten Polyimiden; wodurch eine Schichtbildung und Haftung leichter erfolgen kann. Die altersabhängige Einlagerung in die vorliegenden Schichten kann durch die Vorpolymerisationsdauer bei 150°C beeinflußt werden, wie in Abb. 4 zu sehen ist, denn während der Heizphase bei 150°C können sich daher die Polymerketten räumlich besser nach den Templatmolekülen orientieren.

Ein Aufbringen der Schichten auf massensensitive Transducer wie zum Beispiel Quartz Micro Balance ist leicht möglich und erlaubt damit eine sensorische Anwendung.

## Patentansprüche

1. Ölqualitätssensor, insbesondere für die Prüfung der Qualität von Motorölen, mit einer sensitiven Schicht, die eine durch Prägen mit einem Öl erzeugte speziell an mindestens einen in dem Öl vorliegenden Bestandteil adaptierte Oberfläche oder ein Schichtvolumen hat, die bzw. das entsprechend einer vorliegenden Affinität des Bestandteils zu der Schicht zur spezifischen Wiedereinlagerung dieses Bestandteils prädestiniert ist, **dadurch gekennzeichnet, dass** die sensitive Schicht wenigstens ein Polyimid oder Polyamid umfasst.

2. Ölqualitätssensor nach Anspruch 1, **dadurch gekennzeichnet, daß** das Polyamid beziehungsweise Polyimid der sensitiven Schicht durch Polykondensationsreaktion erhältlich ist ausgehend von wenigstens einem Carbonsäureanhydrid und wenigstens einem zwei- oder mehrfunktionellen Amin.

3. Ölqualitätssensor nach Anspruch 1 oder 2, **dadurch gekennzeichnet, daß** das Polyimid oder Polyamid der sensitiven Schicht erhältlich ist durch Polykondensationsreaktion ausgehend von wenigstens einem mehrfunktionellen Carbonsäureanhydrid und wenigstens einem zwei- oder mehrfunktionellen Amin.

4. Ölqualitätssensor nach einem der Ansprüche 1 bis 3, **dadurch gekennzeichnet, daß** das Polyamid oder Polyimid der sensitiven Schicht erhältlich ist in einer wenigstens zweistufigen Reaktion, wobei in der ersten Reaktionsstufe auf vorzugsweise zwischen 60° und 90° C erwärmt wird zum Starten der Polyamidbildung und weiters dann auf vorzugsweise zwischen 130° und 170° C erhitzt wird zur Entfernung des Lösungsmittels und Vervollständigung der Polyamidbildung und in der zweiten Reaktionsstufe auf Temperaturen über 200° C erhitzt wird um die Polyimide zu bilden.

5. Ölqualitätssensor nach einem der Ansprüche 1 bis 4, **dadurch gekennzeichnet, daß** das Polyamid oder Polyimid der sensitiven Schicht erhältlich ist ausgehend von wenigstens einem mehrfunktionellen Carbonsäureanhydrid, wenigstens einem difunktionellen Amin und wenigstens einem trifunktionellen Amin.

6. Ölqualitätssensor nach einem der Ansprüche 1 bis 5, **dadurch gekennzeichnet, daß** das Polyamid oder Polyimid der sensitiven Schicht erhältlich ist ausgehend von Pyromellitsäuredianhydrid als mehrfunktionelle Carbonsäureanhydrid- Komponente.

7. Ölqualitätssensor nach einem der Ansprüche 1 bis 6, **dadurch gekennzeichnet, daß** das Polyamid oder Polyimid der sensitiven Schicht erhältlich ist ausgehend von Phenylendiamin als difunktionelle Aminkomponente.

8. Ölqualitätssensor nach einem der Ansprüche 1 bis 7, **dadurch gekennzeichnet, daß** das Polyamid oder Polyimid der sensitiven Schicht erhältlich ist ausgehend von wenigstens einem aliphatischen Amin als Aminkomponente.

9. Ölqualitätssensor nach einem der Ansprüche 1 bis 8, **dadurch gekennzeichnet, daß** das Polyamid oder Polyimid der sensitiven Schicht erhältlich ist ausgehend von wenigstens einem trifunktionellen aliphatischen Amin als Aminkomponente.

10. Ölqualitätssensor nach einem der Ansprüche 1 bis 9, **dadurch gekennzeichnet, daß** das Polyamid oder Polyimid der sensitiven Schicht erhältlich ist ausgehend von einem Anteil von etwa 40% bis etwa 80% einer Triaminkomponente.

11. Ölqualitätssensor nach einem der Ansprüche 1 bis 10, **dadurch gekennzeichnet, daß** das Polyamid oder Polyimid der sensitiven Schicht erhältlich ist unter Verwendung eines Überschusses wenigstens einer der Aminkomponenten in der Monomermischung.

12. Ölqualitätssensor nach einem der Ansprüche 1 bis 11, **dadurch gekennzeichnet, daß** das Polyamid oder Polyimid in der sensitiven Schicht erhältlich ist ausgehend von Triaminopyrimidin undloder 4-Aminomethyl-1,8-diaminooktan als trifunktioneller Aminkomponente, die als Vernetzer wirkt.

13. Verfahren zur Herstellung der sensitiven Schicht eines Ölqualitätssensors auf Polyimid- oder Polyamidbasis gemäß einem der Ansprüche 1 bis 12, **dadurch gekennzeichnet, daß** man die Monomerkomponenten mischt, dann das interessierende Öl zugibt und dann diesen Ansatz mindestens so hoch erhitzt, daß die Reaktion startet.

14. Verfahren zur Herstellung der sensitiven Schicht eines Ölqualitätssensors nach Anspruch 13, **dadurch gekennzeichnet, daß** man die nach einer ersten Reaktionszeit entstehende homogene Lösung auf einen für eine FT-IR-Messung geeigneten Preßling aufbringt oder auf einen Schwingquarz aufbringt und danach eine Zeit lang bei einer höheren Reaktionstemperatur in der ersten Reaktionsstufe erhitzt.

15. Verfahren zur Herstellung der sensitiven Schicht eines Ölqualitätssensors nach einem der Ansprüche 13 oder 14, **dadurch gekennzeichnet, daß** man in einer ersten Reaktionsstufe auf eine Temperatur von etwa 60°C bis 90°C erwärmt zum Starten der Amidbildung und weiters auf eine Temperatur von etwa 130° bis 170° C erhitzt zum Entfernen des Lösungsmittels und Vervollständigung der Polyamidbildung.

16. Verfahren zur Herstellung der sensitiven Schicht eines Ölqualitätssensors nach einem der Ansprüche 13 bis 15, **dadurch gekennzeichnet, daß** man in einem weiteren Schritt das Polyamid der sensitiven Schicht auf dem Träger (Preßling oder Schwingquarz) vorzugsweise auf eine Temperatur von etwa 230° bis 270° zur Imidbildung erhitzt.

17. Verfahren zur Herstellung der sensitiven Schicht eines Ölqualitätssensors nach einem der Ansprüche 13 bis 16, **dadurch gekennzeichnet, daß** man zu Beginn eine Lösung der Monomeren in N-Methyl-pyrrolidon herstellt, vorzugsweise in einer Konzentration von zwischen etwa 0,05 mol/l und 0,2 mol/l.

18. Verfahren zur Herstellung der sensitiven Schicht eines Ölqualitätssensors nach einem der Ansprüche 13 bis 17, **dadurch gekennzeichnet, daß** man zu der fertigen Mischung der Monomeren das interessierende Öl in einer bezogen auf die Monomerkomponenten etwa 1,3 bis 1,7-fachen Gewichtsmenge, vorzugsweise in einer etwa 1,5-fachen Gewichtsmenge zugibt.

19. Verwendung eines Ölqualitätssensors nach einem der Ansprüche 1 bis 12 zur Bestimmung der Ölalterung des Öls einer Brennkraftmaschine.

20. Verwendung nach Anspruch 19, **dadurch gekennzeichnet, daß** die Brennkraftmaschine in einem Kraftfahrzeug angeordnet ist und die Bestimmung on-Board erfolgt.

21. Verwendung eines Ölqualitätssensors nach einem der Ansprüche 1 bis 12 als massesensitiven Transducer.

22. Verwendung nach Anspruch 21, **dadurch gekennzeichnet, daß** der Transducer eine Quartz Micro Balance ist.

## Claims

1. Oil quality sensor, especially for testing the quality of motor oils, having a sensitive layer which has a surface or a layer volume which is specially adapted to at least one constituent present in the oil by imprinting with an oil, and is preorganized in accordance with certain affinity of the constituent to the layer for specific reincorporation of this constituent, **characterized in that** the sensitive layer comprises at least one polyimide or polyamide.

2. Oil quality sensor according to Claim 1, **characterized in that** the polyamide or polyimide of the sensitive layer is obtainable by polycondensation reaction starting from at least one carboxylic anhydride and at least one bi- or polyfunctional amine.

3. Oil quality sensor according to Claim 1 or 2, **characterized in that** the polyimide or polyamide of the sensitive layer is obtainable by polycondensation reaction starting from at least one polyfunctional carboxylic anhydride and at least one bi- or polyfunctional amine.

4. Oil quality sensor according to any of Claims 1 to 3, **characterized in that** the polyamide or polyimide of the sensitive layer is obtainable in an at least two-stage reaction, the first reaction stage involving heating to preferably between 60 and 90°C to initiate the polyamide formation and then further heating to preferably between 130 to 170°C to remove the solvent and complete the polyamide formation, and the second reaction stage involving heating to temperatures above 200°C, in order to form the polyimides.

5. Oil quality sensor according to any of Claims 1 to 4, **characterized in that** the polyamide or polyimide of the sensitive layer is obtainable starting from at least one polyfunctional carboxylic anhydride, at least one difunctional amine and at least one trifunctional amine.

6. Oil quality sensor according to any of Claims 1 to 5, **characterized in that** the polyamide or polyimide of the sensitive layer is obtainable starting from pyromellitic dianhydride as the polyfunctional carboxylic anhydride component.

7. Oil quality sensor according to any of Claims 1 to 6, **characterized in that** the polyamide or polyimide of the sensitive layer is obtainable starting from phenylenediamine as the difunctional amine component.

8. Oil quality sensor according to any of Claims 1 to 7, **characterized in that** the polyamide or polyimide of the sensitive layer is obtainable starting from at least one aliphatic amine as the amine component.

9. Oil quality sensor according to any of Claims 1 to 8, **characterized in that** the polyamide or polyimide of the sensitive layer is obtainable starting from at least one trifunctional aliphatic amine as the amine component.

10. Oil quality sensor according to any of Claims 1 to 9, **characterized in that** the polyamide or polyimide of the sensitive layer is obtainable starting from a proportion of from about 40% to about 80% of a triamine component.

11. Oil quality sensor according to any of Claims 1 to 10, **characterized in that** the polyamide or polyimide of the sensitive layer is obtainable using an excess of at least one of the amine components in the monomer mixture.

12. Oil quality sensor according to any of Claims 1 to 11, **characterized in that** the polyamide or polyimide in the sensitive layer is obtainable starting from triaminopyrimidine and/or 4-amino-methyl-1,8-diaminooctane as the trifunctional amine component which acts as a crosslinker.

13. Process for producing the sensitive layer of an oil quality sensor based on polyimide or polyamide according to any of Claims 1 to 12, **characterized in that** the monomer components are mixed, then the oil of interest is added and then this mixture is heated at least to the extent that the reaction commences.

14. Process for producing the sensitive layer of an oil quality sensor according to Claim 13, **characterized in that** the homogeneous solution formed after a first reaction time is applied to a pressed disc suitable for an FT-IR analysis or is applied to an crystal oscillator and afterwards heated in the first reaction stage at a higher reaction temperature for a period.

15. Process for producing the sensitive layer of an oil quality sensor according to either of Claims 13 or 14, **characterized in that** the mixture is heated in a first reaction stage to a temperature of from about 60°C to 90°C to initiate the polyamide formation and further heated to a temperature of from about 130 to 170°C to remove the solvent and complete the polyamide formation.

16. Process for producing the sensitive layer of an oil quality sensor according to any of Claims 13 to 15, **characterized in that**, in a further step, the polyamide of the sensitive layer is heated on the support (pressed disc or crystal oscillator) preferably to a temperature of from about 230 to 270°C for imide formation.

17. Process for producing the sensitive layer of an oil quality sensor according to any of Claims 13 to 16, **characterized in that**, at the beginning, a solution of the monomers in N-methylpyrrolidone is prepared, preferably in a concentration of between about 0.05 mol/l and 0.2 mol/l.

18. Process for producing the sensitive layer of an oil quality sensor according to any of Claims 13 to 17, **characterized in that** the oil of interest is added to the finished mixture of the monomers in an amount of from about 1.3 to 1.7 times the weight, based on the monomer components, preferably in an amount of about 1.5 times the weight.

19. Use of an oil quality sensor according to any of Claims 1 to 12 for determining the oil ageing of the oil of an internal combustion engine.

20. Use according to Claim 19, **characterized in that** the internal combustion engine is disposed in a motor vehicle and the determination is effected on board.

21. Use of an oil quality sensor according to any of Claims 1 to 12 as a mass-sensitive transducer.

22. Use according to Claim 21, **characterized in that** the transducer is a quartz microbalance.

## Revendications

1. Détecteur de qualité d'huile, en particulier pour la vérification de la qualité d'huiles moteur, avec une couche sensible qui présente une surface ou un volume de couche créé par marquage par une huile et adapté spécialement à au moins un composant présent dans l'huile, la surface ou le volume de couche étant destiné à réincorporer spécifiquement ce composant en fonction de l'existence d'une affinité du composant par rapport à la couche, **caractérisé en ce que** la couche sensible comprend au moins un polyimide ou un polyamide.

2. Détecteur de qualité d'huile selon la revendication 1, **caractérisé en ce que** le polyamide ou selon le cas le polyimide de la couche sensible peut être obtenu par une réaction de polycondensation à partir d'au moins un anhydride d'acide carboxylique et d'au moins une amine bifonctionnelle ou polyfonctionnelle.

3. Détecteur de qualité d'huile selon la revendication 1 ou 2, **caractérisé en ce que** le polyimide ou le polyamide de la couche sensible peut être obtenu par une réaction de polycondensation à partir d'au moins un anhydride d'acide carboxylique polyfonctionnel et d'au moins une amine bifonctionnelle ou polyfonctionnelle.

4. Détecteur de qualité d'huile selon l'une des revendications 1 à 3, **caractérisé en ce que** le polyamide ou le polyimide de la couche sensible peut être obtenu par une réaction en au moins deux étapes dans laquelle, dans la première étape de réaction, on chauffe de préférence entre 60° et 90°C pour lancer la formation du polyamide et ensuite à de préférence entre 130° et 170°C pour éliminer le solvant et compléter la formation de polyamide, tandis que dans la deuxième étape de réaction, on chauffe à des températures supérieures à 200°C pour former les polyimides.

5. Détecteur de qualité d'huile selon l'une des revendications 1 à 4, **caractérisé en ce que** le polyamide ou le polyimide de la couche sensible peut être obtenu à partir d'au moins un anhydride polyfonctionnel d'acide carboxylique, d'au moins une amine bifonctionnelle et d'au moins une amine trifonctionnelle.

6. Détecteur de qualité d'huile selon l'une des revendications 1 à 5, **caractérisé en ce que** le polyamide ou le polyimide de la couche sensible peut être obtenu à partir de dianhydride d'acide pyromellitique comme composant de l'anhydride d'acide carboxylique polyfonctionnel.

7. Détecteur de qualité d'huile selon l'une des revendications 1 à 6, **caractérisé en ce que** le polyamide ou le polyimide de la couche sensible peut être obtenu à partir de phénylène diamine comme composant amine bifonctionnelle.

8. Détecteur de qualité d'huile selon l'une des revendications 1 à 7, **caractérisé en ce que** le polyamide ou le polyimide de la couche sensible peut être obtenu à partir d'au moins une amine aliphatique comme composant amine.

9. Détecteur de qualité d'huile selon au moins l'une des revendications 1 à 8, **caractérisé en ce que** le polyamide ou le polyimide de la couche sensible peut être obtenu à partir d'au moins une amine aliphatique trifonctionnelle comme composant amine.

10. Détecteur de qualité d'huile selon l'une des revendications 1 à 9, **caractérisé en ce que** le polyamide ou le polyimide de la couche sensible peut être obtenu à partir d'une proportion d'environ 40% à environ 80% d'un composant triamine.

11. Détecteur de qualité d'huile selon l'une des revendications 1 à 10, **caractérisé en ce que** le polyamide ou le polyimide de la couche sensible peut être obtenu en utilisant un excès d'au moins l'un des composants amine dans le mélange de monomères.

12. Détecteur de qualité d'huile selon l'une des revendications 1 à 11, **caractérisé en ce que** le polyamide ou le polyimide de la couche sensible peut être obtenu à partir de triaminopyrimidine et/ou de 4-aminométhyl-1,8-diaminooctane comme composant amine trifonctionnelle, qui agit comme agent de réticulation.

13. Procédé pour la préparation de la couche sensible d'un détecteur de qualité d'huile à base de polyimide ou de polyamide selon l'une des revendications 1 à 12, **caractérisé en ce que** l'on mélange les composants monomères, **en ce que** l'on ajoute ensuite l'huile concernée et que l'on chauffe ensuite ce mélange à une température suffisamment élevée pour que la réaction commence.

14. Procédé pour la préparation de la couche sensible d'un détecteur de qualité d'huile selon la revendication 13, **caractérisé en ce que** l'on applique la solution homogène obtenue après une première durée de réaction sur une ébauche comprimée qui convient pour une mesure FT-IR ou qu'on l'applique sur un cristal oscillateur, et qu'ensuite on chauffe pendant une certaine durée à une température de réaction supérieure dans la première étape de réaction.

15. Procédé pour la préparation de la couche sensible d'un détecteur de qualité d'huile selon l'une des revendications 13 ou 14, **caractérisé en ce que** dans une première étape de réaction, on chauffe à une température d'environ 60°C à 90°C pour lancer la formation de l'amide et qu'ensuite on chauffe à une température d'environ 130° à 170°C pour éliminer le solvant et compléter la formation du polyamide.

16. Procédé pour la préparation de la couche sensible d'un détecteur de qualité d'huile selon l'une des revendications 13 à 15, **caractérisé en ce que** dans une autre étape, on chauffe le polyamide de la couche sensible présente sur le support (ébauche comprimée ou quartz oscillateur) de préférence à une température d'environ 230° à 270°C pour former l'imide.

17. Procédé pour la préparation de la couche sensible d'un détecteur de qualité d'huile selon l'une des revendications 13 à 16, **caractérisé en ce qu'**au début, on prépare une solution des monomères dans la N-méthylpyrrolidone, de préférence à une concentration comprise entre environ 0,05 mole/l et 0,2 mole/l.

18. Procédé pour la préparation de la couche sensible d'un détecteur de qualité d'huile selon l'une des revendications 13 à 17, **caractérisé en ce qu'**au mélange terminé des monomères, on ajoute l'huile concernée en une quantité en poids qui représente environ 1,3 à 1,7 fois celle des composants monomères, et de préférence à une quantité en poids d'environ 1,5 fois celle-ci.

19. Utilisation d'un détecteur de qualité d'huile selon l'une des revendications 1 à 12, pour la détermination du vieillissement de l'huile d'un moteur à combustion interne.

20. Utilisation selon la revendication 19, **caractérisée en ce que** le moteur à combustion interne est disposé dans un véhicule automobile et **en ce que** la détermination s'effectue à bord.

21. Utilisation d'un détecteur de qualité d'huile selon l'une des revendications 1 à 12 comme transducteur sensible à la masse.

22. Utilisation selon la revendication 21, **caractérisée en ce que** le transducteur est une microbalance à quartz.
